# EUROPEAN PATENT SPECIFICATION

(11) **EP 3 341 719 B1**
(45) Date of publication and mention of the grant of the patent: **25.09.2019**
(21) Application number: 16762940.1
(22) Date of filing: 26.08.2016
(51) Int. Cl.: G01N 29/24, A61B 8/00, B06B 1/02, H01R 4/00

(54) **PROBE ASSEMBLY AND SYSTEM INCLUDING A MODULAR DEVICE AND A CABLE ASSEMBLY**
SONDE UND SYSTEM MIT EINEM MODULAREN GERÄT UND EINER KABELANORDNUNG
ASSEMBLAGE DE SONDE ET SYSTÈME CONTENANT UN DISPOSITIF MODULAIRE ET UNE ASSEMBLAGE DE CÂBLE

(30) Priority: 27.08.2015 US 201514837842
(43) Date of publication of application: 04.07.2018
(73) Proprietor: Creganna Unlimited Company, Ballybrit, Galway (IE)
(72) Inventor: PETERSON, Ryan, Wilsonville, OR 97070 (US); MEDINA, Thomas, J., Portland, OR 97229 (US); WANG, Jian, Fremont, CA 94555 (US); SUN, Jibin, Redwood City, CA 94065 (US)
(74) Representative: Johnstone, Douglas Ian
(86) International application number: PCT/US2016/049068
(87) International publication number: WO 2017/035496

(56) References cited:
- WO-A1-2010/047678
- US-A- 5 957 850
- US-A1- 2009 124 136
- US-B1- 6 645 145

## Description

### CROSS-REFERENCE TO RELATED APPLICATION

The present application includes subject matter that is similar to subject matter described in U.S. Application No. 14/837,895, which was filed on the same day as the present application and is entitled "Array Connector and Method of Manufacturing the Same", which is incorporated herein by reference in its entirety.

### BACKGROUND

There is a general market demand in the electrical and/or optical technology industries to increase data throughput while improving or at least maintaining performance. A countervailing market demand, however, is that the devices or systems that use the electrical and/or optical technology be reduced in size. These market demands are made throughout the consumer electronic industry and medical device industry in which different components communicate with each other electrically or optically. As one example with respect to medical devices, there is a desire to replace catheter-based two-dimensional (2D) intracardiac echo (ICE) imaging systems with real-time three-dimensional (3D) imaging systems. Both of these imaging systems may utilize a catheter having a probe coupled to a distal end of the catheter. The catheter may be configured for insertion into a patient's body (e.g., human or animal). The catheter is communicatively coupled to a user device through a cable assembly. The cable assembly is configured to communicate data signals from the probe to the user device.

US 5,957,850 discloses an ultra-sound transducer probe assembly comprising phased arrays of transducers. The transducer arrays are typically supported on a dielectric substrate including flex circuitry to provide a connection between the transducer arrays and electrical contacts also on the substrate. The probe assembly is connected to a console via a conductor cable. The connection between the electrical contacts of the probe and the cable conductors may be permanent. Alternatively, the electrical contacts supported by the dielectric material of the probe assembly can be provided in the form of a plug member that can be inserted into a receptacle provided at the end of the cable, the electrical contacts of the plug member coupling to corresponding electrical contacts provided in the body of the receptacle.

In order to achieve higher quality imaging and/or 3D imaging, medical device makers have sought to replace the conventional piezoelectric transducer probes with capacitive micromachined ultrasonic transducer (CMUT) probes or piezoelectric micromachined ultrasound transducer (PMUT) probes. The CMUT and PMUT probes may be fabricated using microelectromechanical systems (MEMS) manufacturing techniques.

WO 2010/047678 also discloses an ultra-sound imaging assembly comprising a finger-mountable probe connected to a display station or console via a multi-conductor cable. A transducer assembly, optionally including capacitive micromachined ultrasonic transducers (CMUTs), is connected to flex circuitry provided on a flexible polymeric dielectric material that can be rolled into a cylinder for insertion into a finger-mountable housing. An electrical connector is provided to connect electrical contacts provided on the transducer flex circuitry with the electrical cable.

Although the CMUT and PMUT probes have been demonstrated as being feasible, the CMUT and PMUT probes may be commercially impractical. CMUTs and PMUTs typically include a dense transducer array of sensing elements. For instance, the transducer array may have about 1000 sensing elements/cm². US 6,645, 145 addresses the problem of how to miniaturize the multiplexing of transducer signals via cable connections for diagnostic purposes. US 6,645,145 addresses this problem through the provision of an ultra-sound system comprising arrays of ultra-sound transducers, such as capacitive micro-machined ultrasonic transducers (CMUTs) together with one or more additional components such as micro-relays, micro-switches and inductors. The presence of the micro-switches facilitates multiplexing of the transducer signals without the need for large switching boards.

Communicating data signals that are based on external signals detected by this transducer array can be challenging. For example, it is often desirable or necessary that the catheter and
corresponding cable assembly have a cross-sectional size that is capable of being inserted into a patient's body. Heretofore, a commercially reasonable method for interconnecting the transducer array and the cable assembly while maintaining a reduced cross-sectional size is lacking. A similar problem may also exist in other industries or technologies that utilize a small modular device that is coupled to a cable assembly.

### BRIEF DESCRIPTION

In an embodiment a probe assembly is provided that comprises a modular device comprising an array of elements for detecting external signals or emitting energy, the modular device including a device array having at least one of electrical contacts or optical fiber ends, wherein the array of elements is communicatively coupled to the device array (676). The probe assembly also includes a cable assembly for communicatively coupling the modular device to a computing system and for transmitting data signals there-through. The cable assembly comprises an array connector having a connector body that includes a mating side and channels extending through the mating side and the connector body. The cable assembly includes a plurality of communication lines disposed within corresponding channels of the connector body. The communication lines include at least one of wire conductors or optical fibers and have end faces (415) that are positioned proximate to the mating side to form a terminal array. The terminal array is aligned with and coupled to the device array of the modular device. The connector body includes a plurality of substrate layers that are stacked side-by-side and have respective mating edges that form the mating side. The substrate layers form a plurality of interfaces in which each interface is defined between adjacent substrate layers, wherein the adjacent substrate layers define the channels there-between.

Optionally, the probe assembly includes a probe body that surrounds the modular device and that is configured to be inserted into an individual, such as a human body or animal body. For example, the modular device may include at least one of a capacitive micromachined ultrasonic transducer (CMUT) or a piezoelectric micromachined ultrasonic transducers (PMUT) that is surrounded by the probe body.

Optionally, the communication lines include wire conductors and conductive bumps that are directly coupled to corresponding end faces of the wire conductors. The conductive bumps form corresponding mating terminals and are presented along the mating side to form the terminal array. The conductive bumps are electrically coupled to corresponding electrical contacts of the device array. In some embodiments, the device array is coupled to the terminal array
through one of a thermo-compression bond, a solderless bond, or an anisotropic conductive film or gel.

In an embodiment, a system is provided that includes a probe assembly as defined above. The system also includes a control device that for receiving data signals based on the external signals or for transmitting data signals to the modular device for emitting energy.

### BRIEF DESCRIPTION OF THE DRAWINGS

Figure 1 is a perspective view of an array connector formed in accordance with an embodiment.
Figure 2 is a side view of the array connector of Figure 1.
Figure 3 is a flowchart of a method of manufacturing an array connector in accordance with an embodiment.
Figure 4 illustrates different steps of the method of Figure 3 in greater detail.
Figure 5 is an Scanning Electron Microscope (SEM) image of a cross-section of a working layer formed in accordance with an embodiment.
Figure 6 is another SEM image of the working layer of Figure 5.
Figure 7 is a front end view of a working layer formed in accordance with an embodiment.
Figure 8 is a plan view of the working layer of Figure 7.
Figure 9 is a plan view of a working layer formed in accordance with an embodiment.
Figure 10A is a perspective view of an array connector as substrate layers of the array connector are being stacked in accordance with an embodiment.
Figure 10B is a plan view of a substrate layer in which the communication lines are secured to a coupling layer in accordance with an embodiment.
Figure 10C is a plan view of a substrate layer in which half of the communication lines are secured to a first coupling layer and half of the communication lines are secured to a second coupling layer positioned underneath the first coupling layer.
Figure 11 is a side cross-section of an array connector formed in accordance with an embodiment.
Figure 12 is a side cross-section of the array connector of Figure 11 after a mating side of the array connector has been modified.
Figure 13 is a side cross-section of the array connector of Figure 11 after conductive bumps have been deposited or grown along the mating side.
Figure 14 is an image of an end of a cable assembly formed in accordance with an embodiment.
Figure 15 is an enlarged view of a mating side of the cable assembly.
Figure 16 is a side cross-section of an array connector formed in accordance with an embodiment being coupled to a modular device.
Figure 17 is a side cross-section of an array connector formed in accordance with an embodiment being coupled to a modular device.
Figure 18 illustrates a system formed in accordance with an embodiment that utilizes an array connector.
Figure 19 is a perspective view of a distal end of a probe assembly formed in accordance with an embodiment that utilizes an array connector.
Figure 20 is a partially exploded view of a probe assembly in accordance with an embodiment in which a cable assembly is poised to be coupled to a modular device.
Figure 21 is a perspective view of the probe assembly of Figure 20 with a probe body removed.
Figure 22 illustrates a mating side of an array connector in accordance with an embodiment.
Figure 23 illustrates a mating side of an array connector in accordance with an embodiment.
Figure 24 is a side cross-sectional view of an exemplary electrode that may be used with various embodiments.
Figure 25 is a side cross-sectional view of an exemplary piezoelectric ultrasonic element that may be used with various embodiments.
Figure 26 is a side cross-sectional view of an exemplary CMUT element that may be used with various embodiments.
Figure 27 is a side cross-sectional view of an exemplary PMUT element that may be used with various embodiments.

### DETAILED DESCRIPTION

According to the invention, the array connector includes a plurality of substrate layers that are stacked side-by-side to form, at least in part, the connector body. The communication lines may extend along interfaces between adjacent substrate layers. As used herein, the term "substrate layer" is not limited to a single continuous body of material unless otherwise recited. For example, each substrate layer may be formed form multiple sub-layers of the same or different materials. Moreover, each substrate layer may include one or more features of different materials located therein or extending therethrough. The different substrate layers may be formed using known layer-fabricating processes, such as photolithography, etching, sputtering, evaporation, casting (e.g., spin coating), chemical vapor deposition, electrodeposition, epitaxy, thermal oxidation, physical vapor deposition, and the like. One or more layers may also be formed using a molding process, such as micromolding or nanoimprint lithography (NIL).

As described herein, the mating terminals may form a terminal array that is configured to couple to a corresponding array of another component. Each mating terminal has a fixed location or address with respect to other mating terminals in the terminal array. The terminal arrays may be one dimensional or at least two-dimensional. More specifically, the mating terminals may be positioned in a designated manner along at least two dimensions. For example, the mating terminals may coincide with a plane that extends perpendicular or orthogonal to the communication lines. Alternatively, one or more of the mating terminals may have a different depth or Z-position with respect to other mating terminals. Accordingly, the terminal arrays may be three-dimensional.

A technical effect of at least some embodiments may include the ability to communicate signals to and/or from a dense array of elements. Embodiments may directly connect the cross-sectional end faces of wire conductors (or conductive bumps) to similar terminals. Likewise, embodiments may directly align ends of optical fibers with other optical components. The mating side of the array connectors may include a proud surface that is modified to have designated characteristics. Embodiments may be assembled layer-by-layer to build a 2D or 3D structure of any desired combination of elements. At least some embodiments may enable increased terminal density and product scalability, and reduce assembly cost and product variability.

As used herein, the term "communication line" may include one or more electrical conductors or one or more optical fibers. For example, a communication line may include a single insulated wire conductor or may include two or more insulated wire conductors, such as twin-axial (or twinax) cables. The communication line may also include a coaxial cable. In some embodiments, the communication line only includes the electrical conductor (e.g., wire conductor plus insulation or an uninsulated wire conductor) or the optical fiber. In such embodiments, the end face of the corresponding conductor/fiber may constitute or be part of the mating terminal of the communication line that is used to form an array. In other embodiments, however, the communication line may include a discrete mating terminal that is positioned relative to the end face of the conductor/fiber. For example, the mating terminal may be a conductive bump or plating that is attached to the end face of an electrical conductor or a lens that is positioned adjacent to an optical fiber. Accordingly, the term "mating terminal" may be an end face of a conductor/fiber or a discrete element that is operably coupled to the end face, such as a conductive bump that is attached to the end face of an electrical conductor or a lens that is positioned to transmit optical signals to and/or from a respective optical fiber end.

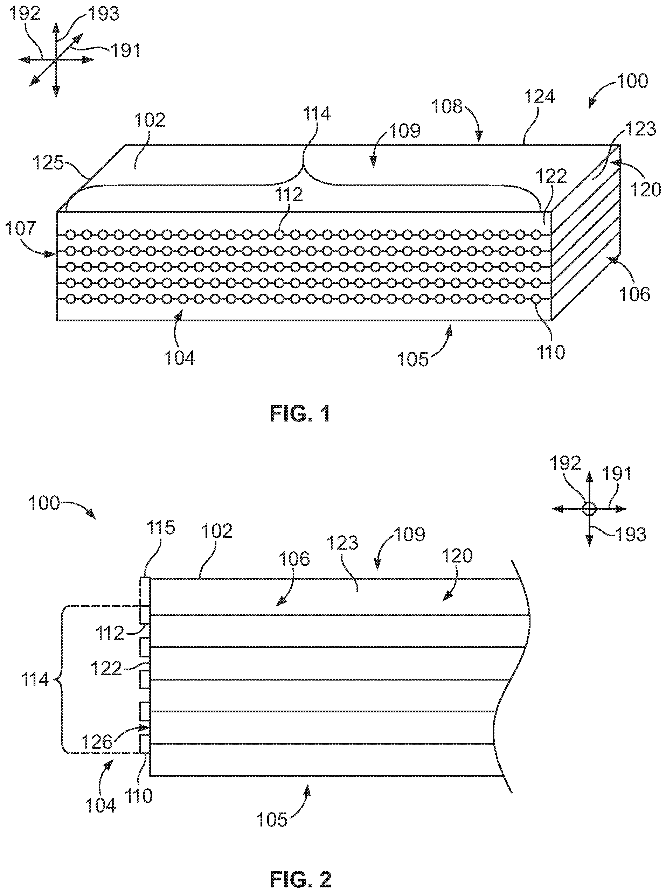

Figure 1 is a perspective view of an array connector 100 formed in accordance with one embodiment, and Figure 2 is a side view of the array connector 100. The array connector 100 is oriented with respect to mutually perpendicular axes, including a mating axis 191, a lateral axis 192, and an elevation axis 193. The array connector 100 has a connector body 102 that includes a plurality of body sides 104-109. The body sides 107 and 108 are not shown in Figure 2. In the illustrated embodiment, each of the body sides 104-109 is a planar side and extends parallel to a plane defined by two of the axes 191-193. In other embodiments, however, one or more of the body sides 104-109 are not planar and/or do not extend parallel to a plane defined by two of the axes 191-193. The body side 104 is configured to interface with another component to communicatively couple the array connector 100 and the other component. As such, the body side 104 is hereinafter referred to as the mating side 104.

The array connector 100 has a plurality of communication lines 110 that extend through the connector body 102. The communication lines 110 may include one or more wire conductors and/or one or more optical fibers. In particular embodiments, the communication lines 110 are wire conductors. As shown, the communication lines 110 include mating terminals 112 that are exposed along the mating side 104. The mating terminals 112 may include or be end faces of the corresponding wire conductors or optical fibers or may be discrete elements that are positioned relative to the end faces of the corresponding wire conductors or optical fibers. The communication lines 110 extend from the mating side 104 and through the connector body 102 such that the communication lines 110 either clear the connector body 102 or have corresponding mating terminals (not shown) that are exposed along one of the other body sides 105-108. In particular embodiments, the communication lines 110 extend entirely through the connector body 102 and clear the connector body 102 such that the communication lines 110 project away from the connector body 102. In some embodiments, the communication lines 110 may be grouped or bundled together to form one or more cables or cable harnesses (not shown).

In the illustrated embodiment, the connector body 102 is substantially rectangular or block-shaped in which each body side is opposite another body side. In other embodiments, however, the connector body 102 may have any one of a variety of shapes. For example, the connector body 102 may have a trapezoidal shape in which the mating side 104 has a smaller area than the opposite side 108. In such embodiments, the communication lines 110 may flare away from each other as the communication lines 110 extend from the mating side 104 to the body side 108.

In some embodiments, the mating terminals 112 are ends of the segments of the communication lines 110 that extend through the connector body 102. For example, the mating terminals 112 may be ends of optical fibers that are positioned proximate to the mating side 104. In such embodiments, the mating terminals 112 may project from the mating side 104, as shown in Figure 2. Alternatively, the mating terminals 112 may be end faces that are flush with the mating side 104 or may be located a small depth within the connector body 102. In particular embodiments, however, the mating terminals 112 are formed from material that is deposited or grown at the end faces of wire conductors. For example, the mating terminals 112 may constitute metal bumps or platings that are formed through solder dispensing, solder screen printing, electroplating, electrolessplating, physical vapor deposition (PVD), or the like. As shown in Figure 2, the mating terminals 112 may project a bump distance 115 away from the mating side 104. Alternatively, the conductive bumps may be flush with mating side 104.

The mating terminals 112 form a terminal array 114 in which each mating terminal 112 has a designated location or address relative to the other mating terminals 112. In the illustrated embodiment, the terminal array 114 includes a plurality of columns and rows of mating terminals 112. It should be understood, however, that the locations of the mating terminals 112 in the terminal array 114 may be arranged in a different manner based upon the application of the array connector 100.

In particular embodiments, the terminal array 114 of mating terminals 112 forms a high-density array. As used herein, a "high-density array" includes at least 50 mating terminals per 100 mm² or at least 75 mating terminals per 100 mm². In some embodiments, the high density array may have at least 100 mating terminals per 100 mm², at least 200 mating terminals per 100 mm², at least 300 mating terminals per 100 mm², or at least 400 mating terminals per 100 mm². In particular embodiments, the high density array may have at least 500 mating terminals per 100 mm² or at least 750 mating terminals per 100 mm². In more particular embodiments, the high density array may have at least 1000 mating terminals per 100 mm².

In particular embodiments, the terminal array 114 is a two-dimensional array such that the mating terminals 112 coincide with a common plane. In other words, the mating terminals 112 may be coplanar. For example, the terminal array 114 coincides with a plane that extends parallel to the elevation axis 193 and the lateral axis 192. In other embodiments, however, the terminal array 114 may not coincide with a common plane. For example, each row of mating terminals 112 may have alternating positions along the mating axis 191.

As described herein, the connector body 102 may comprise a plurality of substrate layers 120 that are stacked side-by-side. Each substrate layer 120 may have a plurality of layer edges 122-125. In order to distinguish the different layer edges, the layer edges 122 may be referred to as leading edges or mating edges. The layer edges 124 (Figure 1) may be referred to as trailing edges or loading edges, and the layer edges 123, 125 may be referred to as side edges. In the illustrated, the communication lines 110 extend parallel to the side edges 123, 125 and perpendicular to the leading and trailing edges 122, 124.

The mating edges 122 collectively form the mating side 104 when the substrate layers 120 are stacked side-by-side along the elevation axis 193. In the illustrated embodiment, the mating edges 122 are even or flush with one another such that the mating side 104 has a mating surface 126 that is essentially planar. The mating terminals 112 project away from the mating surface 126. Yet in other embodiments, the mating edges 122 of the substrate layers 120 may not be even or flush with one another. For example the mating edges 122 may form a stair-like structure in which each mating edge 122 has a different location along the mating axis 191.

Accordingly, the array connector 100 presents a mating side 104 that is capable of being interconnected with a corresponding array of another component. Each of the substrate layers 120 may be shaped such that adjacent substrate layers 120 form channels (not shown) that extend between the adjacent substrate layers 120. The stacked substrate layers 120 may form a substantially monolithic body that holds segments of the communication lines 110 in fixed positions with respect to one another. For example, the connector body 120 may consist essentially of the communication lines 110, the substrate layers 120, and the mating terminals 112 if the mating terminals 112 comprise a material that differs from the communication lines 110.

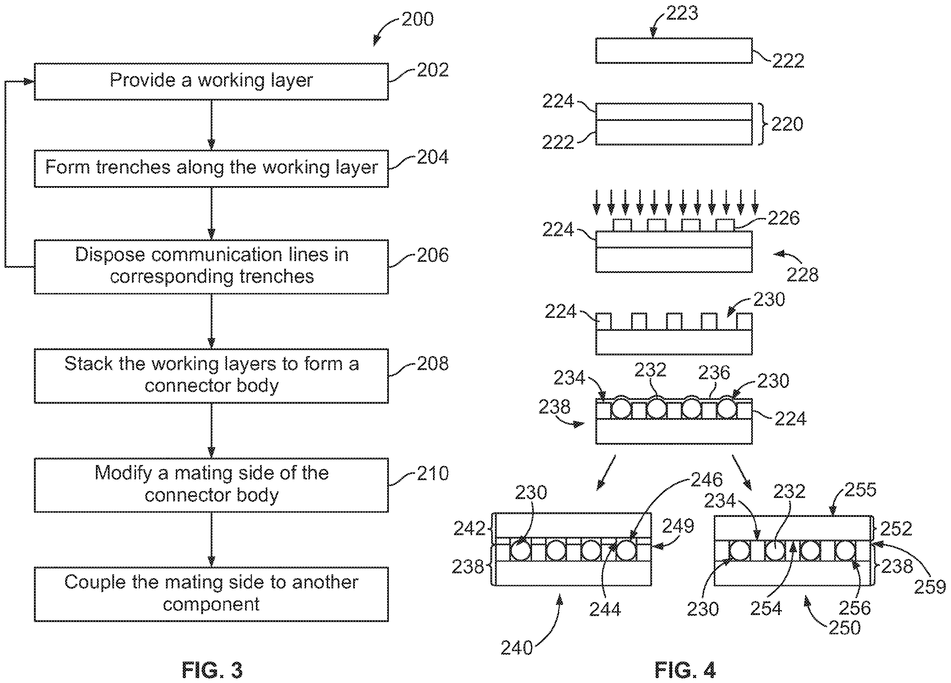

Figure 3 is a flowchart of a method 200 of manufacturing an array connector in accordance with an embodiment. The array connector may be similar or identical to, for example, the array connector 100 shown in Figure 1. The method 200 may employ structures or aspects of various embodiments described herein. In various embodiments, certain steps may be omitted or added, certain steps may be combined, certain steps may be performed simultaneously, certain steps may be performed concurrently, certain steps may be split into multiple steps, certain steps may be performed in a different order, or certain steps or series of steps may be re-performed in an iterative fashion.

The method 200 may include a plurality of additive or subtractive steps in which working layers (or portions thereof) are added or subtracted, respectively, from a working substrate. The terms "working layer" and "working substrate" are used to describe intermediate objects that are used to form an array connector, such as the array connector 100. More specifically, the term "working layers" includes one or more layers of material that may be used to form a substrate layer. For example, the term encompasses a single base layer and a base layer having a photoresist deposited thereon. The term "working substrate" includes a plurality of stacked substrate layers in which at least one of the substrate layers is being used to form an array connector. For example, in some cases, the term may encompass an array connector prior to the mating side being modified.

The following describes only one method of manufacturing an array connector. It should be understood that the method may be modified or that other methods may be used to manufacture the array connectors. At least one of the substrate layers may be formed using one or more processes that are similar to, for example, the processes used to manufacture integrated circuits, semiconductors, and/or microelectromechanical systems (MEMS). For example, lithography (e.g., photolithography) is one category of techniques or processes that may be used to fabricate the array connectors described herein. Exemplary lithographic techniques or processes are described in greater detail in Marc J. Madou, Fundamentals of Microfabrication and Nanotechnology: Manufacturing Techniques for Microfabrication and Nanotechnology, Vol. II, 3rd Edition, Part I (pp. 2-145), which is incorporated herein by reference in its entirety.

One or more processes for fabricating the substrate layers and/or the array connectors may include subtractive techniques in which material is removed from a working substrate. In addition to lithography, such processes include (1) chemical techniques, such as dry chemical etching, reactive ion etching (RIE), vapor phase etching, chemical machining (CM), anisotropic wet chemical etching, wet photoetching; (2) electrochemical techniques, such as electrochemical etching (ECM), electrochemical grinding (ECG), photoelectrochemical etching; (3) thermal techniques, such as laser machining, electron beam machining, electrical discharge machining (EDM); and (4) mechanical techniques, such as physical dry etching, sputter etching, ion milling, water-jet machining (WJM), abrasive water-jet machining (AWJM), abrasive jet machining (AJM), abrasive grinding, electrolytic in-process dressing (ELID) grinding, ultrasonic drilling, focused ion beam (FIB) milling, and the like. The above list is not intended to be limiting and other subtractive techniques or processes may be used. Exemplary subtractive techniques or processes are described in greater detail in Marc J. Madou, Fundamentals of Microfabrication and Nanotechnology: Manufacturing Techniques for Microfabrication and Nanotechnology, Vol. II, 3rd Edition, Part II (pp. 148-384), which is incorporated herein by reference in its entirety.

One or more processes for fabricating the substrate layers and/or the array connectors may also include additive techniques in which material is added to a working substrate. Such processes include PVD, evaporation (e.g., thermal evaporation), sputtering, ion plating, ion cluster beam deposition, pulsed laser deposition, laser ablation deposition, molecular beam epitaxy, chemical vapor deposition (CVD) (e.g., atmospheric pressure CVD (APCVD), low pressure CVD (LPCVD), very low pressure CVD (VLPCVD), ultrahigh vacuum CVD (UHVCVD), metalorganic CVD (MOCVD), laser-assisted chemical vapor deposition (LACVD), plasma-enhanced CVD (PECVD), atomic layer deposition (ALD), epitaxy (e.g., liquid-phase epitaxy, solid-phase epitaxy), anodization, thermal spray deposition, electroplating, electroless plating, incorporation in the melt, thermal oxidation, laser sputter deposition, reaction injection molding (RIM), spin coating, polymer spraying, polymer dry film lamination, casting, plasma polymerization, silk screen printing, ink jet printing, mechanical microspotting, microcontact printing, stereolithography or microphotoforming, nanoimprint lithography, electrochemical forming processes, electrodeposition, spray pyrolysis, electron beam deposition, plasma spray deposition, micromolding, LIGA (which is a German acronym for x-ray lithography, electrodeposition, and molding), compression molding, and the like. The above list is not intended to be limiting and other additive techniques or processes may be used. Exemplary additive techniques or processes are described in greater detail in Marc J. Madou, Fundamentals of Microfabrication and Nanotechnology: Manufacturing Techniques for Microfabrication and Nanotechnology, Vol. II, 3rd Edition, Part III (pp. 384-642), which is incorporated herein by reference in its entirety.

In some cases, one or more processes may provide array connectors with identifiable physical characteristics. For example, channels formed within the array connector may be identified as etched channels or molded channels based upon inspection of the array connector. More specifically, a scanning electron microscope (SEM) or other imaging system may capture an image of the array connector, such as a sliced portion of the array connector. The channels may have qualities or characteristics that are indicative of surfaces that are etched or molded.

The method 200 is described with reference to other Figures of the present application. The method 200 includes providing, at 202, a working layer 220 (shown in Figure 4). The working layer 220 may be any suitable material for fabricating an array connector as described herein. For example, the working layer 220 includes a base layer 222 and a channel layer 224 coupled to the base layer 222. The channel layer 224 is suitable for having select portions of the channel layer 224 removed. For example, the channel layer 224 may comprise an etchable material (e.g., organic material).

In some cases, the base layer 222 may undergo surface modification to enhance adhesion of the channel layer 224 to the base layer 222. For example, a top surface 223 of the base layer 222 may be subjected to silanization. In the illustrated embodiment, the base layer 222 includes glass (e.g., silicon wafer), and the channel layer 224 includes photoresist, such as a negative photoresist. In particular embodiments, the photoresist is SU-8. SU-8 includes Bisphenol A Novolac epoxy that is dissolved in an organic solvent (gamma-butyrolactone GBL or cyclopentanone, depending on the formulation) and up to 10 wt% of mixed Triarylsulfonium/hexafluoroantimonate salt as the photoacid generator. Upon irradiation, the photoacid generator decomposes to form hexafluoroantimonic acid that protonates the epoxides on the oligomer. The protonated oxonium ions are available to react with neutral epoxides in a series of cross-linking reactions after application of heat. Each monomer molecule contains eight reactive epoxy sites, and therefore high degree of cross-linking can be obtained after photothermal activation giving a negative tone. This results in high mechanical and thermal stability of the lithographic structures after processing. It is contemplated, however, that materials other than SU-8 may be used in alternative embodiments, such as other photoresists.

At 204, the method 200 may include forming trenches 230 within the channel layer 224. For example, a mask 226 may be applied to the channel layer 224 and the resulting working layer 228 may be subjected to an ultraviolet (UV) exposure for a designated amount of time. The UV exposure may form the trenches 230 within the channel layer 224. In alternative embodiments, the trenches may be formed through additive techniques. Methods of working with and patterning SU-8 are also described in del Campo, Aranzazu, and Christian Greiner. "SU-8: a photoresist for high-aspect-ratio and 3D submicron lithography." Journal of Micromechanics and Microengineering 17.6 (2007): R81; Abgrall, Patrick, et al. "SU-8 as a structural material for labs-on-chips and microelectromechanical systems." Electrophoresis 28.24 (2007): 4539-4551; Lee, Jeong Bong, Kyung-Hak Choi, and Koangki Yoo. "Innovative SU-8 Lithography Techniques and Their Applications." Micromachines 6.1 (2014): 1-18, each of which is incorporated herein by reference.

At 206, the method 200 may include disposing communication lines 232 within corresponding trenches 230. As shown in Figure 4, the communication lines 232 have a height that clears an outer surface 234 of the channel layer 224. In other embodiments, the communication lines 232 have heights that are flush with the outer surface 234 or do not clear the outer surface 234 such that the communication lines 232 are located at a depth within the trenches 230.

Optionally, an adhesive 236 may be applied to the working layer 238. The adhesive 236 may be, for example, an epoxy. The adhesive 236 may be deposited along the outer surface 234 and/or within the trenches 230. The adhesive 236 may at least partially fill voids formed between the communication line 232 and the corresponding surfaces that define the trench 230. The adhesive 236 may facilitate securing the communication lines 232 in essentially fixed positions with respect to other elements of the working layer 238. In particular embodiments, the adhesive 236 is a silane adhesion promoter that couples glass or a silicon wafer to SU-8. For example, the adhesive 236 may may be applied using the Gelest method. In particular embodiments, the adhesive 236 is 1,3-bis(3-Glycidoxypropyl)TetramehylDisiloxane.

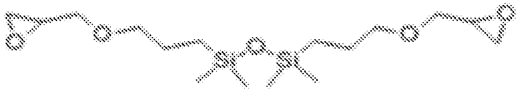

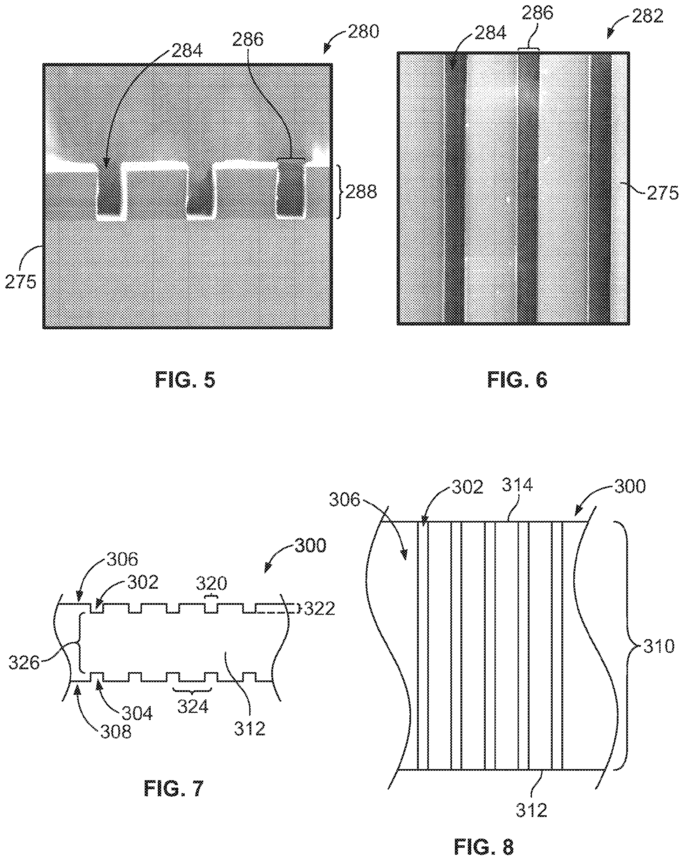

At 208, the working layers may be stacked onto one another to form a connector body. Figure 4 demonstrates two different embodiments in which the working layers have been stacked to form a corresponding connector body. More specifically, a connector body 240 and a connector body 250 are shown in Figure 4. The connector body 240 may be formed by stacking a second working layer 242 onto the first working layer 238. The second working layer 242 may have trenches 244 along one side that faces the first working layer 238. In some embodiments, the second working layer 242 has trenches on both sides of the second working layer 242. The second side (or the side that faces away from the first working layer 238) may have communication lines disposed within the trenches. An example of such a working layer is shown in Figure 7.

As the second working layer 242 is lowered onto the first working layer 238, the surfaces that define the trenches 244 may engage the communication lines 232 disposed within the trenches 230 of the first working layer 238. In such embodiments, the communication lines 232 may cause the second working layer 242 to align with the first working layer 238 to form a plurality of channels 246 extending through the connector body 240. Accordingly, each channel 246 is formed by one of the trenches 230 and one of the trenches 244.

Similarly, the connector body 250 may be formed by stacking a second working layer 252 onto a first working layer 238. The second working layer 252, however, may be devoid of trenches along a side 254 of the second working layer 252 that faces the first working layer 238. Optionally, the second working layer 252 may include trenches along a side 255 that is opposite the side 254. The communication lines 232 may be flush with the outer surface 234 or located a depth within the trenches 230. When the second working layer 252 is lowered onto the first working layer 238, the side 254 of the second layer 252 engages the outer surface 234 of the first layer 238 and covers the trenches 230 to form a plurality of channels 256.

In both connector bodies 240, 250, the first and second working layers are adjacent substrate layers that define interfaces 249, 259, respectively, therebetween. In each example, the adjacent substrate layers of each interface 249, 259 are shaped to form the channels 246, 256, respectively. In each example, the communication lines 232 are disposed within corresponding channels 246, 256 of the connector body such that the communication lines 232 extend along the corresponding interface.

Although Figure 4 only illustrates two working layers being stacked side-by-side with each other, the method 200 may include repeatedly stacking numerous layers side-by-side. As described herein, the working layers may have trenches formed along one or both sides of the working layers. The communication lines may have ends that are proximate to the corresponding mating edges of the substrate layers. The ends may form a terminal array or be subsequently modified to form the terminal array, such as the terminal array 114.

The following describes one particular example of a method of manufacturing the substrate layers, such as the method 200 (Figure 3), in which the substrate layer includes sixty-four (64) trenches having a maximum trench width of 75 ± 5 µm that is measured between opposing side surfaces that define the corresponding trench and a maximum trench depth of 37.5 ± 5 µm that is measured from the outer surface of the substrate layer to the bottom of the corresponding trench. The trenches may have a center-to-center spacing (or pitch) of 240 µm pitch. The substrate layer may have total thickness of 480 µm that is measured from one outer surface to an opposite outer surface.

The trenches may be formed using photolithographic coating and etching of SU-8 based photoresist. SU-8 is a negative photoresist that has been used directly as a structural material due to its mechanical strength, chemical resistance, and thermal stability. The SU-8 can be cross-linked upon UV exposure.

One or both sides of the substrate layer (or working layer) may be processed to include the trench. The process included preparing a working substrate for forming the trenches. Preparing the working substrate included subjecting a silicon wafer to oxygen plasma treatment to clean and activate the surface of the silicon wafer (APE 110 Plasma Chamber, 150 Watts RF Power, <0.30 Torr Vacuum, 2 minute residence time, 2 cycles, 125 sccm Gas Flow). This activated surface was then subjected to a surface modification process. More specifically, a surface silanization process may be conducted after the surface has been activated by the oxygen plasma treatment. The silanization enhances adhesion of SU-8 to the silicon wafer. The silanization process included: adjusting pH of 95% Ethanol-5% DI H2O mixture to ∼5 with dilute acid; adding 2 ml silane in 100 ml mixture of the aqueous alcohol and stir; allowing 5 minutes for hydrolysis and silanol formation; immersing wafer for 2 minutes; rinsing with ethanol and air drying; curing wafer for 10 minutes at 110°C on a hotplate. Adhesion was enhanced due to the bonding of the silane functional group with the silicon wafer coupled with the presence of epoxy ring in both silane and SU-8. In some embodiments, a silane adhesion promoter may be used to couple SU-8 of one working layer to the silicon wafer of another working layer.

The process also included film deposition that included dynamic dispensing via quick injection with syringe containing 4g Microchem SU-8-305 followed by spincoating with 2000rpm-ramp 300 seconds. The target thickness (75 ± 1µm) was obtained when ambient is -21.7°C. The spin-speed program varies with room temperature, equipment, and lab conditions.

The process also included baking the working substrate, also referred to as a soft bake. For instance, progressive heating with ramp from room temperature to 95°C for 20 minutes may be beneficial to reduce intrinsic stress.

After the baking, the working substrate was exposed to UV light having a wavelength of 365 nm. The prescribed exposure dose may range from 150 to 250 mJ/cm2 to attain ∼75 µm thickness. A target energy level of 200 mJ/cm2 may be determined using a dosimeter. After exposing the working substrate, the working substrate was baked at 65°C for 1 minute and then 95°C for 5 minutes. The working substrate was then immersed in propylene glycol methyl ether acetate (PGMEA), double puddle, minimum 4 minutes each, with moderate agitation, and then rinsed with fresh PGMEA. The working substrate was then baked (i.e., hard baked) at 150°C for 30 minutes for permanent structural integrity. The working substrate was then diced to generate multiple working substrates having trenches formed thereon.

Figures 5 and 6 include SEM images 280, 282, respectively, of an exemplary working substrate 275 with trenches 284 that were manufactured using a process that was similar to the process described above. In Figures 5 and 6, the trenches 284 have a trench width 286 and a trench depth or height 288. The trench width 286 is about 77.5 µm and the trench depth 288 is about 105.0 µm. Although only a single side of the working substrate has trenches therealong, trenches may be formed along both sides of the working substrate. Alternatively, two separate working substrates each having trenches on one side may be coupled side-by-side to form a composite working substrate having trenches on both sides.

As another example, working substrates (or layers) may be formed using a photostructurable glass ceramic (PSGC). PSGC may enable forming microstructures, such as the trenches, therein without use of any of conventional drilling or machining processes. For example, a wafer may be exposed to a designated UV light for a predetermined period of time using a mask. The exposed regions may be converted to a ceramic material by baking at a designated temperature for a predetermined time. More specifically, the PSGC may transform into the crystalline phase lithium metasilicate. The transformed material may be more active for reaction with hydrofluoric acid (HF) than amorphous glass. In this manner, the trenches may be formed in the working layers.

Figures 7 and 8 illustrate a front end view and a top-down view, respectively, of a portion of a working layer 300 having trenches 302, 304 prior to communication lines being disposed within the trenches 302 and trenches 304 (Figure 7). The working layer 300 has first and second layer sides 306, 308 (Figure 7). The trenches 302 are located along the first layer side 306, and the trenches 304 are located along the second layer side 308. In some embodiments, the working layer 300 may be fabricated from a single working layer in which both layer sides are subject to a trench-forming process (e.g., etching). Alternatively, the working layer 300 may be formed from two separate working sub-layers in which each working sub-layer has one planar side and an opposite side with trenches therealong. The two planar sides may be coupled to each other to form the working layer 300 shown in Figures 7 and 8.

The trenches 302, 304 are open-sided channels or grooves that extend an entire axial dimension 310 (Figure 8) of the working layer 300. The working layer 300 includes a mating edge 312 and a trailing edge 314 (Figure 8) with the axial dimension 310 defined therebetween. The trenches 302, 304 extend the entire axial dimension 310 such that the trenches 302, 304 extend through the leading and trailing edges 312, 314.

As shown in Figure 7, each of the trenches 302, 304 has a trench width 320 and a trench depth 322. The trenches 302, 304 has have a lateral center-to-center spacing (or pitch) 324 and an elevated center-to-center spacing (or pitch) 326. The trench width 320, the trench depth 322, the center-to-center spacing 324, and the elevated spacing 326 may have a range of values. For example, the trench width 320 and the trench depth 322 may be configured to hold only a single communication line (e.g., single wire conductor or single optical fiber). In other embodiments, the trench width 320 and the trench depth 322 may be configured to hold multiple communication lines. For example, the trench width 320 and the trench depth 322 may be configured to hold a differential pair of wire conductors. The communication lines may have, for example, an American Wire Gauge (AWG) between 30 AWG to 50 AWG. A diameter of the communication lines may be from about 0.30 mm to about 0.01 mm.

By way of example only, the trench width 320 may be less than or equal to 250 µm, less than or equal to 150 µm, less than or equal to 125 µm, or less than or equal to 100 µm. In particular embodiments, the trench width 320 may be less than or equal to 90 µm, less than or equal to 80 µm, or less than or equal to 70 µm. In more particular embodiments, the trench width 320 may be less than or equal to 60 µm, less than or equal to 50 µm, or less than or equal to 40 µm. By way of example only, the trench depth 322 may be less than or equal to 200 µm, less than or equal to 175 µm, or less than or equal to 150 µm. In particular embodiments, the trench depth 322 may be less than or equal to 130 µm, less than or equal to 110 µm, or less than or equal to 100 µm. In more particular embodiments, the trench depth 322 may be less than or equal to 80 µm, less than or equal to 60 µm, or less than or equal to 40 µm.

The lateral center-to-center spacing 324 may be less than or equal to 1000 µm, less than or equal to 800 µm, or less than or equal to 600 µm. In particular embodiments, the lateral center-to-center spacing 324 may be less than or equal to 500 µm, less than or equal to 400 µm, or less than or equal to 300 µm. The elevated center-to-center spacing 326 may be less than or equal to 1000 µm, less than or equal to 800 µm, or less than or equal to 600 µm. In particular embodiments, the elevated center-to-center spacing 326 may be less than or equal to 500 µm, less than or equal to 400 µm, or less than or equal to 300 µm. In more particular embodiments, the elevated center-to-center spacing 326 may be less than or equal to 250 µm, less than or equal to 200 µm, or less than or equal to 150 µm. In the illustrated embodiment of Figures 7 and 8, the lateral center-to-center spacing 324 is about 240 µm and the elevated center-to-center spacing 326 is about 480 µm.

In the illustrated embodiment, the trenches 302, 304 have identical dimensions (e.g., the trench depth 322 and the trench width 320) and spacings with respect to one another. It should be understood that the dimensions and spacing are not required to be identical. For example, while some trenches 302 may be configured to receive 32 AWG communication lines, other trenches 302 may be configured to receive 50 AWG communication lines. Likewise, the lateral center-to-center spacings 324 and the elevated center-to-center spacing 326 are not required to be the same.

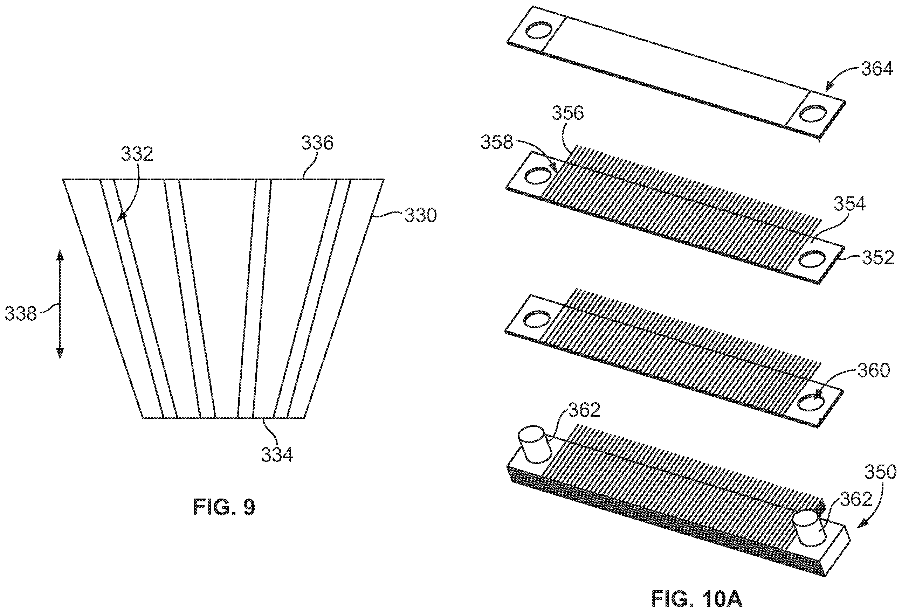

In the embodiment shown in Figures 7 and 8, the trenches 302, 304 have linear paths that extend parallel to the mating axis (not shown), such as the mating axis 191 (Figure 1), and extend parallel to one another. It is contemplated, however, that other embodiments may include paths that are non-linear and/or paths that do not extend parallel to one another. For example, Figure 9 illustrates a top-down view of a working layer 330 that includes a plurality of trenches 332. The working layer 330 has a mating edge 334 and a loading edge 336 that face in opposite directions along a mating axis 338. As shown, the trenches 332 do not extend parallel to one another and are not parallel to the mating axis 338. As the trenches 332 extend from the mating side 334 to the loading edge 336, the trenches 332 may extend or flare away from each other. Such embodiments may be used to effectively change a density of the array on one side of the connector body. For example, the mating edge 334 may collectively form, with other mating edges, a mating side (not shown) having a first terminal array (not shown). The loading edge 336 may collectively form, with other loading edges, a body side (not shown) having a second terminal array (not shown). The first terminal array may have a greater density of mating terminals than the second terminal array.

Figure 10A is a perspective view of a partially-formed array connector 350 as substrate layers 352 of the array connector 350 are being stacked side-by-side onto each other. As shown, each of the substrate layers 352 includes a layer body 354 and a plurality of communication lines 356. The layer body 354 may include one or more sub-layers, as described above, that are stacked together and processed to form the substrate layer 352. The layer body 354 includes trenches 358 and alignment holes 360. The trenches 358 receive segments of the communication lines 356. The alignment holes 360 are configured to receive fixtures 362 of an assembly stage (not shown). The alignment holes 360 and the fixtures 362 may cooperate in aligning the substrate layers 352 with respect to one another. As described above, one or more of the layer bodies 354 may be coated with an adhesive to facilitate securing the substrate layers 352 to one another as the substrate layers 352 are stacked onto each other. A final substrate layer 364 may be stacked on top of the last substrate layer with trenches 358.

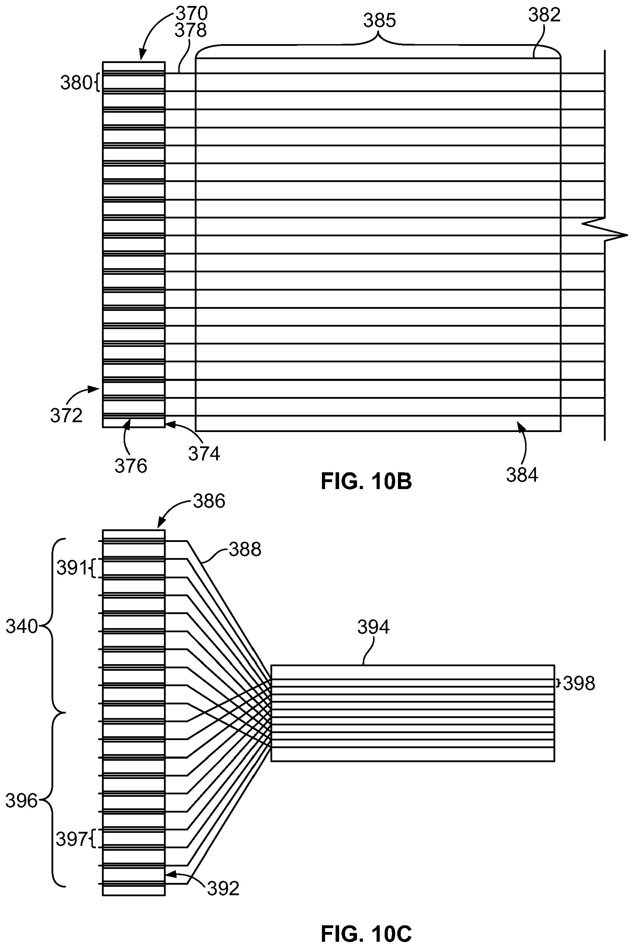

Figure 10B is a plan view of a substrate layer 370 that may have similar or identical features as the substrate layers described herein. The substrate layer 370 includes a mating edge 372, a loading edge 374, and a plurality of trenches or channels 376 extending therebetween. A plurality of communication lines 378 are disposed within corresponding trenches 376. As shown, the communication lines 378 have a center-to-center spacing 380 as the communication lines 378 extend through the corresponding trenches 376. The center-to-center spacing 380 is uniform throughout the substrate layer 370.

The communication lines 378 clear the loading edge 374. In an exterior of the substrate layer 370, the communication lines 378 are secured to a coupling layer 382. In the illustrated embodiment, the coupling layer 382 is a strip of tape having an adhesive outer surface 384. The communication lines 378 are positioned onto the adhesive outer surface 384 and pressed into the adhesive outer surface 384 thereby securing the communication lines 378 to the coupling layer 382. The communication lines 378 may have any designated arrangement. For example, the communication lines 378 are coplanar and have the same center-to-center spacing 380 in Figure 10B throughout the coupling layer 382. In other embodiments, the coupling layer 382 may hold the communication lines 378 at a different center-to-center spacing 380. Yet in other embodiments, the communication lines 378 may be positioned to cross over each other such that the communication lines 378 have different relative positions along the substrate layer 370 than along the coupling layer 382.

Although the coupling layer 382 may be a strip of tape in some embodiments, the coupling layer 382 may also be an overmold in other embodiments. For example, the communication lines 378 may be held at designated positions with respect to one another within a molding cavity. A moldable material (e.g., thermoplastic) may be injected into the molding cavity and allowed to cure to form the overmold. Yet in other embodiments, the coupling layer 382 may include multiple sub-layers. For example, a first sub-layer may include a base layer, such as polyimide. After the communication lines 378 are positioned onto the base layer, an adhesive material may be applied onto the base layer (e.g., sprayed) and allowed to cure thereby forming a second sub-layer and securing the communication lines 378 to the coupling layer 378.

In the illustrated embodiment, the coupling layer 382 has a length (or sub-length) 385 that extends along only a portion of the length of the communication lines 378. In some embodiments, one or more other coupling layer(s) (not shown) may secure the communication lines 378 along a different portion(s) of the length. Although Figure 10B only illustrates one single substrate layer 370 and corresponding coupling layer 382, additional substrate layers 370 may be stacked onto each other as described herein. In such embodiments, the corresponding coupling layers 382 may also be stacked onto each other.

Figure 10C is a plan view of a substrate layer 386 having a plurality of communication lines 388. As shown, a first portion 390 of the communication lines 388 have a first center-to-center spacing 391 through the substrate layer 386. The first portion 390 of communication lines 388 clear a loading edge 392 and attach to a first coupling layer 394. The first coupling layer 394 may be similar to the coupling layer 382. A second portion 396 of the communication lines 388 have a second center-to-center spacing 397 through the substrate layer 386. In the illustrated embodiment, the first and second center-to-center spacings 391, 397 are equal, but may be different in other embodiments. The second portion 396 of the communication lines 388 clear the loading edge 392 and attach to a second coupling layer (not shown) that is positioned below the first coupling layer 394. When stacked onto each other, the first and second coupling layers and corresponding communication lines 388 may have a thickness or height that is equal to or less than a thickness or height of the substrate layer 386. As shown, the communication lines 388 have a third center-to-center spacing 398 through the coupling layer 394 that is less than the first center-to-center spacing 391 and less than the second center-to-center spacing 397. However, the communication lines 388 maintain their relative positions. In such embodiments, the communication lines 388 may occupy a reduced cross-sectional area as the communication lines 388 extend a length of a cable. Although Figure 10C illustrates one method of reducing the cross-sectional area as the communication lines 388 extend between two end points, other methods may be implemented.

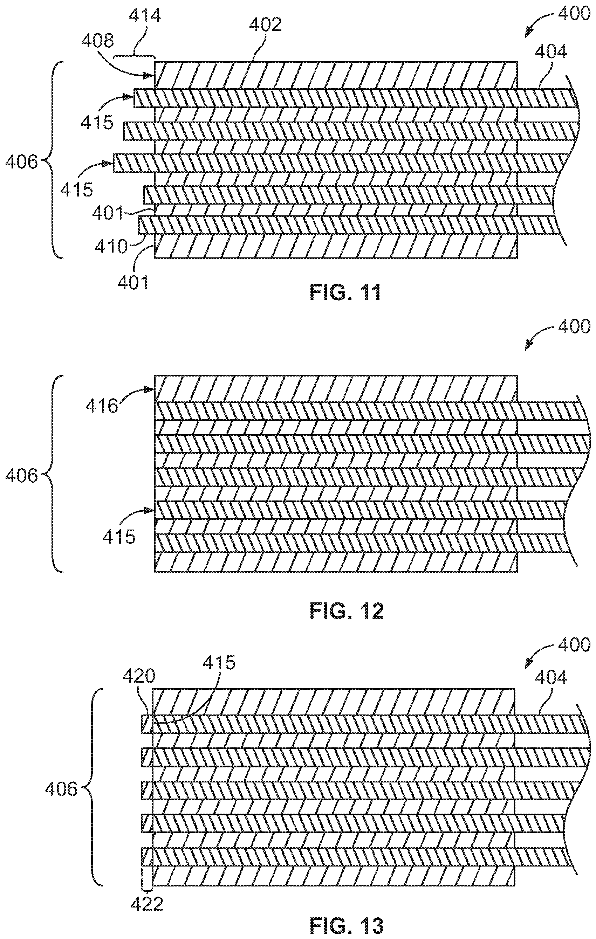

Figures 11-13 illustrate a side cross-section of an array connector 400 at different manufacturing stages. The method 200 (Figure 3) may also include modifying, at 210, a mating side of the connector body to, for example, prepare the mating side for coupling to an array of another component. Figures 11-13 illustrate one example of modifying the mating side. The array connector 400 is formed from a plurality of stacked substrate layers 401 as described herein. As shown in Figure 11, the array connector 400 includes a connector body 402 and a plurality of communication lines 404. The array connector 400 also includes a mating side 406 that is formed from edges surfaces 408 of the corresponding substrate layers 401. The array connector 400 has a plurality of segment projections 410 of the communication lines 404 that extend away from the corresponding edge surfaces 408. The segment projections 410 represent portions of the communication lines 404 that clear or project beyond the edge surface 408 of the corresponding substrate layer 401. The segment projections 410 may exist, for example, after the disposing process and/or after the communication lines 404 are cut.

Each segment projection 410 has a length 414 that is measured between an end face 415 of the corresponding segment projection 410 and the corresponding edge surface 408. The lengths 414 of the segment projections 410 may be different due to tolerances in the method of manufacturing the array connector 400. For some applications, it may be desirable to have a limited planarity requirement with respect to the mating terminals. More specifically, it may be desirable for the end faces 415 of the communication lines 404 to have a common length 414.

Accordingly, modifying the mating side, at 210, may include polishing the mating side 406 to remove the segment projections 410 of the communication lines 404. The array connector 400 after the polishing operation is shown in Figure 12. The polishing operation may include mechanical polishing in which a rough surface is repeatedly driven over the mating side 406. Alternatively or in addition to mechanical polishing, the polishing operation may include other forms of surface modification, such as chemical modification.

The polishing operation may not only remove the segment projections 410 (Figure 11), but may also remove a small portion of the edge surfaces 408 such that a side surface 416 of the mating side 406 is planar. The end faces 415 are coplanar with the side surface 416. In some embodiments, the array connector 400 shown in Figure 12 does not undergo any further modification prior to being coupled to another component. In such embodiments, the end surfaces 415 may form the corresponding mating terminals of the array connector 400. However, in other embodiments, the array connector 400 undergoes at least one other modification operation to, for example, provide conductive bumps to the communication lines.

Figure 13 illustrates the array connector 400 after conductive bumps 420 have been added to the communication lines 404. The conductive bumps 420 may constitute the mating terminals of the array connector 400. In particular embodiments, the conductive bumps 420 are formed from material that is deposited or grown on the end faces 415 of the communication lines 404. For example, the conductive bumps 420 may be formed through solder dispensing, solder screen printing, electroplating, electrolessplating, physical vapor deposition (PVD), or the like. The conductive bumps 420 may comprise, for example, at least one of nickel (Ni), tin (Sn), gold (Au), or other precious metal. The conductive bumps 420 may be formed in a controlled manner to achieve a designated height or length 422 relative to the side surface 416.

In an exemplary embodiment, the height 422 is less than or equal to 100 µm and has a tolerance limit of ± 10 µm. However, the height 422 may have other values with different tolerance limits. For example, the height 422 may be less than or equal to 200 µm, less than or equal to 150 µm, or less than or equal to 125 µm. In particular embodiments, the height 422 may be less than or equal to 110 µm, less than or equal to 100 µm, or less than or equal to 90 µm. In more particular embodiments, the height 422 may be less than or equal to 80 µm, less than or equal to 70 µm, less than or equal to 60 µm, or less than or equal to 50 µm. The tolerance limit may be within ± 15% of the height, within ± 12% of the height, within ± 10% of the height, or within ± 8% of the height.

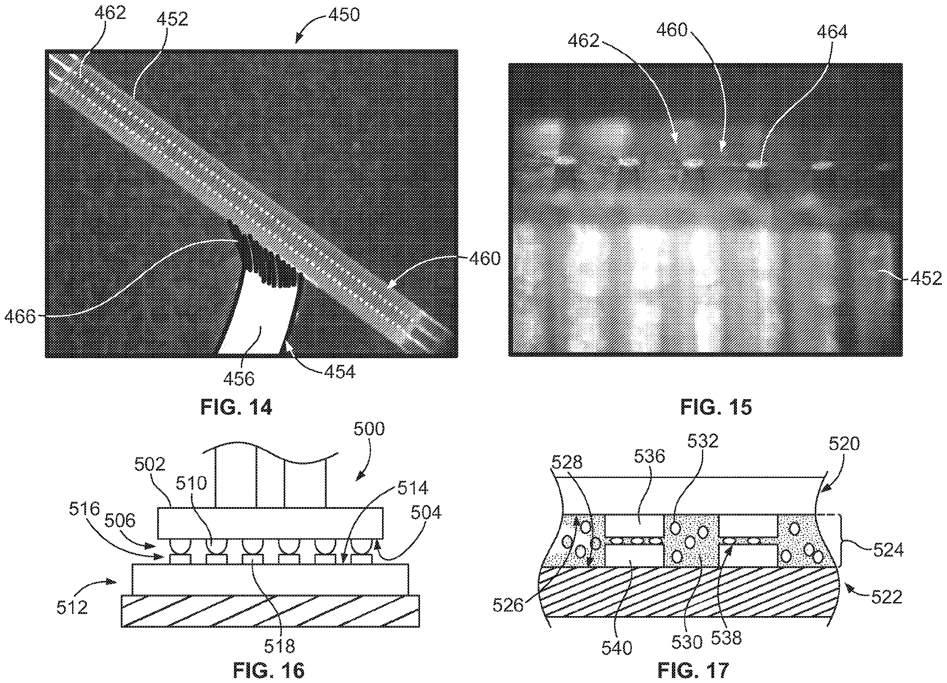

Figure 14 is a perspective view of a cable assembly 450 formed in accordance with an exemplary embodiment, and Figure 15 is an enlarged view of a mating side 460 of the cable assembly 450. The cable assembly 450 includes an array connector 452 and a cable harness 454 (Figure 14) that is communicatively coupled to the array connector 452. The array connector 452 has a mating side 460 that includes a 2 X 64 array of mating terminals 462. As shown in Figure 15, the mating terminals 462 are formed from conductive bumps 464.

The cable harness 454 is configured to group or bunch a plurality of communication lines 466 (Figure 14) together. For example, the cable harness 454 includes a jacket 456 that surrounds each of the communication lines 466. The communication lines 466 project through a body side (not shown) of the array connector 452. The jacket 456 may be formed over the communication lines 466 through an extrusion process, molding process, or wrapping process. During the wrapping process, tape may be helically wrapped about the bundle of communication lines 466. Optionally, the jacket 466 may include a shield layer that surrounds the communication lines 466. In alternative embodiments, the cable harness 454 may include multiple jackets 456.

The method 200 may also include coupling, at 212 (Figure 3), the mating side to another component. More specifically, the mating terminals of the terminal array may be aligned with corresponding terminals of another array and, for some embodiments, the mating terminals and corresponding terminals may be directly coupled. Figures 16 and 17 illustrate two different methods for coupling a terminal array of an array connector to a device array of a modular device. Figure 16 schematically shows a portion of a system having a cable assembly 500 that includes an array connector 502 having a mating side 504. A terminal array 506 is located along the mating side 504 and may include a high density array of mating terminals 510. In an exemplary embodiment, the mating terminals 510 are conductive bumps. The system also includes a modular device 512 having a mounting side 514 that includes a device array 516 of mating terminals 518. In the illustrated embodiment, the mating terminals are electrical contacts (e.g., contact pads) 518 formed along the mounting side 514. The electrical contacts 518 may be electrically coupled to other elements of the modular device 512 through traces and vias.

In the illustrated embodiment, the device array 516 and the terminal array 506 are communicatively coupled through thermocompression flip-chip bonding or thermonsonic flip-chip bonding (also referred to as solderless bonding). In thermocompression flip-chip bonding, the mating terminals 510 of the array connector 502 are bonded to the mating terminals 518 of the modular device 512 by thermal energy and applied force. The bonding temperature may be relatively high, e.g., 300°C, to soften bonding material and increase the diffusion bonding process. In thermosonic (or solderless) flip-chip bonding, the ultrasonic energy is transferred to the bonding joint through the array connector 502. The ultrasonic energy may soften the bonding material and make it vulnerable to plastic deformation. It should be understood that the method of bonding may be identified through inspection. For example, an SEM image of a device may reveal that the device array and terminal array are thermocompression bonded or thermosonic bonded.

Figure 17 is a side schematic view of a system after an array connector 520 has been communicatively coupled to a modular device 522. Prior to the coupling operation, a conductive material 524 may be applied to a mating side 526 of the array connector 520 and/or a mounting side 528 of the modular device 522. The conductive material 524 may be an anisotropic conductive film or gel that includes an adhesive material 530 having conductive particles 532 suspended and/or distributed therein. During the coupling operation, mating terminals 536 of the array connector 520 may interface with corresponding mating terminals 540 of the modular device 522. More specifically, the mating terminals 536 may be electrically coupled to the corresponding mating terminals 540 through the conductive material 524. As shown in Figure 17, conductive bridges 538 are selectively formed through the conductive particles 532 of the conductive material 524.

It should be understood that a terminal array of fiber ends may also be communicatively coupled to a device array of fiber ends. For example, the mating sides of two optical ferrules may have respective arrays of optical fiber ends or lenses that are coupled to optical fiber ends. One optical ferrule may be an array connector as described herein. The other optical ferrule may be similar to a multi-fiber MT ferrule. Optionally, the mating sides may include physical alignment features that engage one another to align the two ferrules. The mating sides may be operably coupled to one another to maintain the alignment throughout operation. For example, the two ferrules may be secured to each other using a fastener or an adhesive.

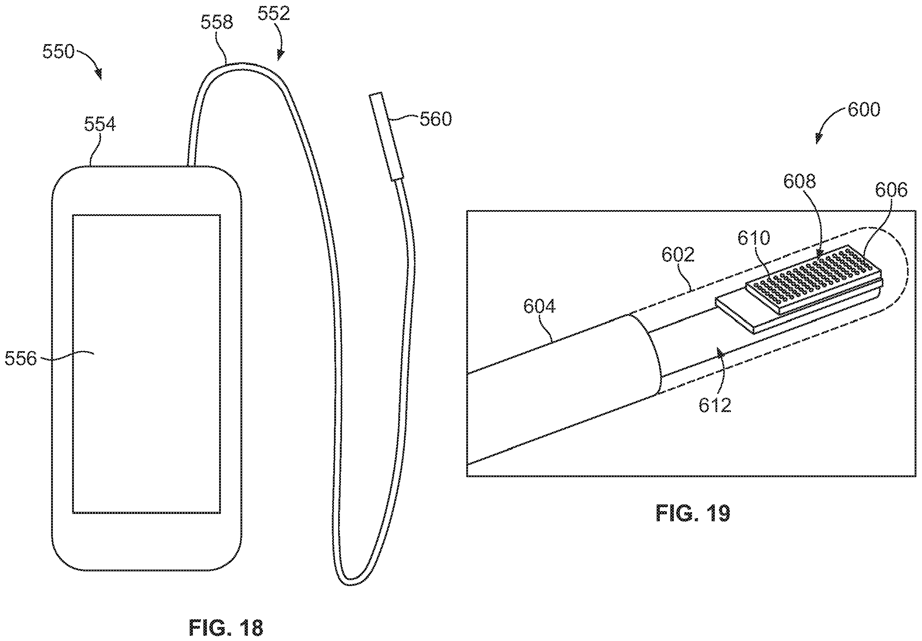

Figure 18 illustrates a system 550 formed in accordance with an embodiment that includes a probe assembly 552 and a control device 554 that are communicatively coupled to one another. In the illustrated embodiment, the control device 554 is a portable user device having a display 556. For example, the control device 554 may be a smartphone or similar handheld communication device. In other embodiments, the control device 554 may be a tablet computer or laptop computer. Yet in other embodiments, the control device 554 may be a larger computing system, such as a workstation. The control device 554 (or computing system) may include one or more processors (or processing units) that are configured to execute program instructions. For example, the control device 554 may receive data signals that are based on external signals detected by the probe assembly 552, process the data signals, and generate useful information for the user. The control device 554 may transform the data signals into images that are shown on the display 556. The display 556 may include a touch screen that is configured to receive user inputs such that a user may control operation of the system 550 through the touch screen. Alternatively or in addition to the touchscreen, the control device 554 may include an input device, such as a keyboard or touchpad, for receiving user inputs. The control device 554 may also be configured to communicatively couple to an external input device, such as a mouse or external keyboard. In some embodiments, the control device 554 may transmit signals to emit energy from a modular device 560 of the probe assembly 552.

In an exemplary embodiment, the probe assembly 552 is a catheter that is configured to be inserted into a body (e.g., human or animal). For example, the probe assembly 552 may be configured for real-time three-dimensional (3D) ultrasound imaging. Ultrasound can be excited by many different methods, including the piezoelectric effect, magnetostriction, and the photoacoustic effect. The probe assembly 552 may also be configured to emit energy for delivering therapy, such as tissue ablation. As shown, the probe assembly 552 includes a cable assembly 558. The cable assembly 558 may include an array connector (not shown), such as the array connectors described herein, and a plurality of communication lines (not shown) that are communicatively coupled to the array connector.

The probe assembly 552 may also include a modular device 560 that is communicatively coupled to the control device 554 through the cable assembly 558. In particular embodiments, the modular device 554 includes a solid state device, such as complementary metal-oxide semiconductors (CMOSs), charge-coupled devices (CCDs), and the like. The modular device 560 may be sized for insertion into, for example, a patient's body. In some embodiments, the modular device 560 is configured to detect or observe external signals. In the illustrated embodiment, the modular device is an ultrasound device or transducer 560. For example, the ultrasound device 560 may be or include a piezoelectric micromachined ultrasonic transducer (PMUT) or a capacitive micromachined ultrasonic transducer (CMUT). In other embodiments, the modular device 560 may include or constitute an imaging sensor (e.g., CMOS). The modular device 560 may also be configured to measure conditions within a designated space, such as pressure or temperature. In some embodiments, the modular device 560 may be configured for providing therapy, such as tissue ablation. Ablation may refer to the direct application of chemical or thermal therapies to a designated region of an organ or tissue in an attempt to at least substantially damage or destroy the designated region. For example, the modular device 560 may be configured to ablate tissue through high intensity focused ultrasound (HIFU), radio-frequency (RF), microwaves, laser, or thermal control (e.g., thermal ablation or cryoablation). The modular device 560 may also be configured for stimulation by delivering electrical pulses. It should be understood that the modular device 560 may also be configured for both detection and therapy in some embodiments.

In some embodiments, the entire system 500 may be configured for insertion into a patient's body. For example, the probe assembly 552 may include a stimulation device (e.g., neurostimulator or pacemaker) and the control device 554 may be a pulse generator that is configured to provide a designated sequence of electrical pulses to the probe assembly 552 for delivering the therapy. The modular device 560 may be, for example, a percutaneous lead or a paddle lead. The control device 554 and the probe assembly 552 may be implanted into a patient's body.

The probe assembly 552, however, may be used for purposes other than medical applications. For example, the modular device 560 may include an imaging sensor (e.g., CMOS) or other type of detector/transducer that detects external signals and communicates the external signals, directly or indirectly, to the control device 554.

Figure 19 is a perspective view of a distal end of a probe assembly 600 in accordance with an embodiment. The probe assembly 600 may be similar or identical to the probe assembly 552 (Figure 18). The probe assembly 600 includes a probe body 602 that is coupled to a cable 604. The probe body 602 is indicated by dashed lines so that internal components may be viewed. The probe body 602 may surround or encapsulate a modular device 606 that is disposed within an interior of the probe body 602. As shown, the modular device is an ultrasound device 606, such as a PMUT or CMUT that includes an array 608 of elements 610. The array 608 may be similar to an array of piezoelectric elements incorporated by conventional ultrasound devices. The array 608 may be a dense array of elements 610. For example, the array 608 may have about 1000 elements/cm². The elements 610 are communicatively coupled to a device array of electrical contacts (not shown), such as the device array 516 (Figure 16).

The array 608 of elements 610 are configured to detect external signals or, more specifically, ultrasound signals from within a region-of-interest (ROI), such as a region within a patient's body. In particular embodiments, the ROI is within a vessel or, more specifically, a cardiac vessel. The modular device 606 is configured to communicate data signals that are based on the ultrasound signals to a computing system. The data signals may be identical to the detected ultrasound signals or may be processed in a predetermined manner by the modular device 606. To this end, the modular device 606 is communicatively coupled to a cable assembly 612, which may be similar or identical to the cable assemblies described herein. For example, the cable assembly 612 may include an array connector (not shown) and wire conductors (not shown) that are coupled to the array connector. The array connector is communicatively coupled to the modular device 606. In alternative embodiments, the elements 610 may be replaced with elements that are configured to detect other external signals and/or are configured to emit energy. For example, the elements 610 may include electrodes for delivering radiofrequency (RF) energy to a designated tissue. In other embodiments, the elements 610 may be electrodes that are configured to apply electrical pulses to a designated tissue. In other embodiments, the elements 610 are configured to deliver HIFU to a designated tissue.

In addition to a device array (not shown) and the array 608, the modular device 606 may include other components. For example, the modular device 606 may include circuitry that is configured to process data signals that are received from the array 608 and/or circuitry that is configured to process data signals that are received from a control device. In some embodiments, the modular device 606 may include a signal converter (or optical engine) that changes the signals between one signal form (e.g., optical) and another signal form (e.g., electrical). The signal converter may be similar to, for example, the engines developed by TE Connectivity and sold under the trademark Coolbit. Accordingly, the modular device 606 may be configured to (a) receive optical signals and/or electrical signals from the cable assembly or (b) provide optical signals and/or electrical signals to the cable assembly.

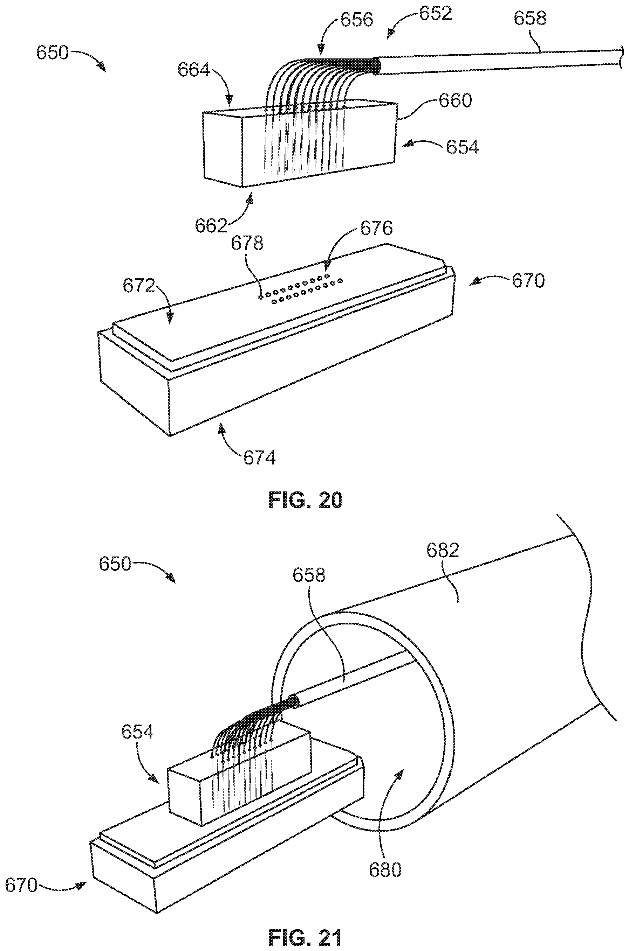

Figures 20 and 21 illustrate a probe assembly 650 during different assembly stages. Figure 20 is a perspective view of a cable assembly 652 having an array connector 654 and a bundle of communication lines 656 coupled to the array connector 654. The communication lines 656 are surrounded and grouped together by a jacket 658 having a circular cross-section. In other embodiments, the jacket 658 may have different cross-sectional dimensions. For example, the jacket 658 may have a ribbon shape.

The array connector 654 may be similar or identical to the array connectors described herein. For example, the array connector 654 includes a connector body 660 having a mating side 662 and a loading side 664. The communication lines 656 extend through the loading side 664 and toward the mating side 662. The communication lines 656 may extend along channels that are formed through the connector body 660. The communication lines 656 may form an array along the mating side 662 such that end faces of wire conductors or optical fibers (not shown) are exposed along the mating side 662 or such that conductive bumps or lenses (not shown) are aligned with the end faces along the mating side 662.

In the illustrated embodiment, the loading side 664 and the mating side 662 face in opposite directions. In other embodiments, however, the loading side 664 and the mating side 662 may face in different directions that are, for example, perpendicular to each other. In such embodiments, the connector body 660 may include channels that are non-linear. For embodiments that have optical fibers, the bending of the optical fibers may satisfy the bend radius for communicating optical signals.

The array connector 654 is configured to be communicatively coupled to a modular device 670. The modular device 670 includes a mounting side 672 and an active side 674. The modular device 670 may be manufactured using, for example, semiconductor or integrated circuit manufacturing technology or microelectromechanical systems (MEMS) manufacturing technology. The modular device 670 may be manufactured using, for example, the subtractive or additive processes described above. The active side 674 includes an array of elements (not shown) that are configured to detect external signals and/or emit energy therefrom. The array of elements are communicatively coupled (e.g., through vias, conductive traces, optical fibers, and/or the like) to a device array 676 positioned along the mounting side 672. The device array 676 includes an array of terminals 678. In some embodiments, the array terminals include electrical contacts 678. The electrical contacts 678 may be, for example, contact pads that are positioned substantially flush with the mounting side 672 or flexible contact beams that project away from the mounting side 672. In some embodiments, the array terminals include optical fiber ends 678 that are exposed along the mounting side 672 for aligning with corresponding optical fiber ends. In some embodiments, the device array 676 includes both electrical contacts and optical fiber ends. The device array 676 is configured to match the array (not shown) along the mating side 662 of the array connector 670.

Figure 21 illustrates the array connector 654 mounted to the modular device 670. The array connector 654 and the modular device 670 may be mechanically and communicatively coupled to each other using, for example, the bonding processes described herein. In other embodiments, the array connector 654 and the modular device 670 are secured to each other without disposing a material between the mating side 662 and the mounting side 672. For example, a fastener may be used to hold the array connector 654 and the modular device 670 in fixed positions with respect to one another. Also shown in Figure 21, the jacket 658 may extend through a passage 680 formed by a sheath 682. For embodiments that are inserted into a patient's body, the sheath 682 may comprise any suitable material that is approved for the desired application. Although not shown, the probe assembly 650 may also include a probe body that is coupled to the sheath 682. The probe body may surround and protect the modular device 670 and the array connector 654. The probe body may be similar to, for example, a cap that is coupled to an end of the sheath 682.

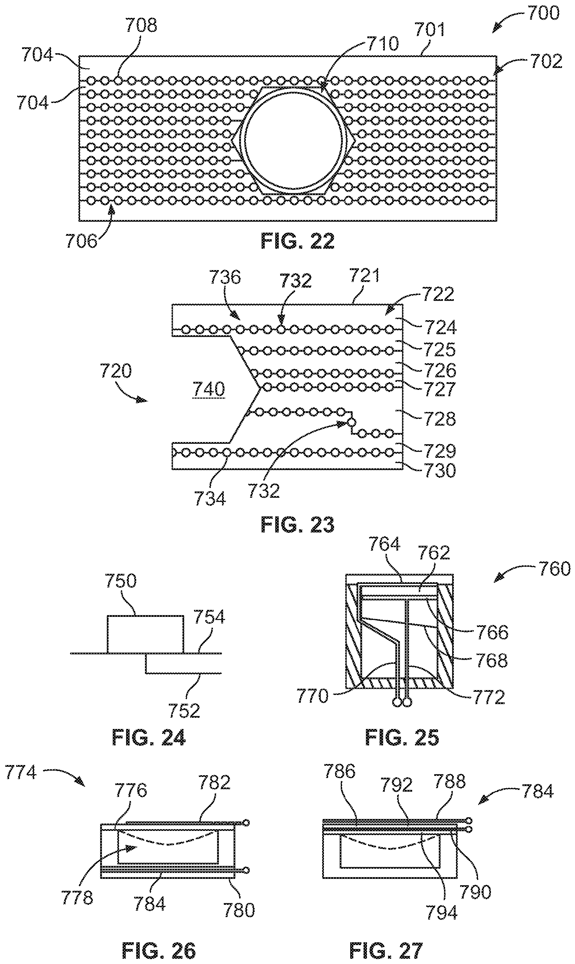

Figure 22 is a plan view of a mating side 702 of an array connector 700 formed in accordance with an embodiment. The array connector 700 may be similar to the array connector 100 (Figure 1) or other array connectors described herein. For example, the array connector 700 has a connector body 701 that includes a plurality of substrate layers 704 that are stacked side-by-side. The substrate layers 704 form a plurality of interfaces 706 in which each interface 706 is defined between adjacent substrate layers 704. The adjacent substrate layers 704 are shaped to form channels therebetween that receive corresponding communication lines 708. The communication lines 708 have end faces that may form an array along the mating side 702. Alternatively, the end faces may be coupled to conductive bumps or lenses of the communication lines.

Also shown, the connector body 701 may have a working passage or channel 710 therethrough. The working passage 710 may be aligned with a corresponding passage of, for example, a modular device (not shown). The working passage 710 and the optional passage of the modular device may be sized and shape to receive an instrument or tool. For example, the working passage 710 and the device passage may be sized and shaped to receive a tube 712. A fluid may be directed through the tube 712 to, for example, remove debris. In other embodiments, the modular device may be disposed within a flexible container or bladder. The tube 712 may provide a fluid along the array of the modular device.

Figure 23 is a plan view of a mating side 722 of an array connector 720 formed in accordance with an embodiment. The array connector 720 may be similar to the array connector 100 (Figure 1) or other array connectors described herein. For example, the array connector 720 has a connector body 721 that includes a plurality of substrate layers 724-730 that are stacked side-by-side. The substrate layers 724-730 form a plurality of interfaces 732 in which each interface 732 is defined between adjacent substrate layers 724-730. The adjacent substrate layers 724-730 are shaped to form channels therebetween that receive corresponding communication lines 734. The communication lines 734 have end faces that may form an array 736 along the mating side 722. Alternatively, the end faces may be coupled to conductive bumps or lenses of the communication lines that form the array 730. Also shown, the array connector 720 includes a working passage 740 therethrough. The working passage 740 may be configured to receive an instrument or tool. Alternatively, the working passage 740 may be shaped to facilitate connecting the array connector 720 to another component.

Figure 23 illustrates that a variety of arrays 736 may be formed. As shown, the substrate layers 724-730 of the array connector 720 have varying thicknesses. For example, the substrate layers 725 and 726 are substantially planar and have substantially equal thicknesses, except for the portions that define the working passage 740. The substrate layer 727 has a substantially uniform thickness that is less than the thicknesses of the other substrate layers. The substrate layer 728 has a non-planar body that has two different thicknesses. In such embodiments, the interfaces 732 may have non-planar contours. For example, the interface 732 between adjacent substrate layers 728 and 729 includes two horizontal sections that are joined by a vertical section. Multiple communication lines 734 are disposed along the horizontal sections and one communication line 734 is disposed along the vertical section. Although Figure 23 illustrates an interface 732 with horizontal and vertical sections, it is understood that non-orthogonal sections may also be formed. For example, a sloping section may extend between the two horizontal sections of the interface 732 between the substrate layers 728, 729.

In the illustrated embodiments, each of the communication lines has only a single communication pathway. In other embodiments, however, the communication lines may include multiple communication pathways. Alternatively, the channels may be sized and shaped to receive more than one communication line. For example, the communication line may include a twin-axial communication line in which two wire conductors extend parallel to each other through a common jacket. As another example, the communication line may comprise a coaxial line.

Figures 24-27 provide exemplary elements that may form the arrays along the modular devices. For example, Figure 24 illustrates an electrode 750 that is electrically coupled to a communication pathway 752 through, for example, a printed circuit 754. Figure 25 illustrates a piezoelectric ultrasonic element 760. The element 760 includes piezoelectric material 762 sandwiched between high conductivity electrode layers 764, 766, which may comprise, for example, gold or platinum. The electrode layer 766 is supported by a backing layer 768. The electrode layers 764, 766 are electrically coupled to conductors 770, 772, respectively.

Figure 26 illustrates a CMUT element 774 that includes a metallized suspended membrane 776 (e.g., silicon nitride (SiₓN_{y})) that is disposed over a cavity 778. The CMUT element 774 also includes rigid substrate 780. When a DC voltage is applied between two electrodes 782, 784, the membrane 776 is deflected, being attracted toward the substrate by electrostatic forces. The mechanical restoring force caused by the stiffness of the membrane 776 resists the attraction. Consequently, ultrasound can be generated from the oscillations of the membrane 776 with an AC voltage input.

Figure 27 illustrates a PMUT element 784 that includes a membrane 786 sandwiching between electrode layers 788, 790. Deflection of the membrane 786 in the PMUT element 784 is caused by lateral strain generated from the piezoelectric effect of the membrane 786. The membrane 786 includes at least one piezoelectric layer 792 and a passive elastic layer 794. In operation, the resonant frequency of the PMUT does not directly depend on the thickness of the piezoelectric layer 792. Instead, the flexural mode resonant frequencies are closely related to the shape, dimensions, boundary conditions, intrinsic stress and mechanical stiffness of membrane. The elements of Figures 25-27 are described in Qiu et al., "Piezoelectric Micromachined Ultrasound Transducer (PMUT) Arrays for Integrated Sensing, Actuation and Imaging" Sensors (2015), which is incorporated herein by reference in its entirety for the purpose of understanding the elements of Figures 25-27.

## Claims

1. A probe assembly (650) comprising:
a modular device (670) comprising an array of elements for detecting external signals or emitting energy, the modular device (670) including a device array (676) having at least one of electrical contacts or optical fiber ends, wherein the array of elements is communicatively coupled to the device array (676); and
a cable assembly (652) for communicatively coupling the modular device (670) to a computing system (554) and transmit data signals therethrough, the cable assembly (652) comprising an array connector (654) having a connector body (660) that includes a mating side (662) and channels (246) extending through the mating side (662) and the connector body (660), the cable assembly (652) including a plurality of communication lines (656) disposed within corresponding channels (246) of the connector body (660), the communication lines (656) including at least one of wire conductors or optical fibers, the communication lines (656) having respective end faces (415) that are positioned proximate to the mating side (662) to form a terminal array (114), the terminal array (114) being aligned with and coupled to the device array (676) of the modular device (670);
wherein the connector body (660) includes a plurality of substrate layers (120) that are stacked side-by-side and have respective mating edges (122) that form the mating side (662), the substrate layers (120) forming a plurality of interfaces (249) in which each interface is defined between adjacent substrate layers (120), wherein the adjacent substrate layers (120) define the channels (246) therebetween.

2. The probe assembly (650) of claim 1, further comprising a probe body (602) for insertion into a body, wherein the probe body (602) surrounds the modular device (670).

3. The probe assembly (650) of claim 2, wherein the modular device (670) includes an ultrasound device that includes at least one of a capacitive micro-machined ultrasonic transducer (CMUT) or a piezoelectric micro-machined ultrasonic transducers (PMUT).

4. The probe assembly (650) of claim 1, wherein the communication lines (656) include wire conductors and conductive bumps (420) directly coupled to corresponding end faces (415) of the wire conductors, the conductive bumps (420) forming corresponding mating terminals (112) and being presented along the mating side (662) to form the terminal array (114), the conductive bumps (420) being electrically coupled to corresponding electrical contacts (678) of the device array (676), preferably wherein the conductive bumps (420) have a height that is less than or equal to 100 µm and has a tolerance limit that is within ± 10 µm.

5. The probe assembly (650) of claim 1, wherein the channels (246) are at least one of etched channels (246) or molded channels (246).

6. The probe assembly (650) of claim 1, wherein the terminal array (114) includes at least 50 mating terminals (112) per 100 mm².

7. The probe assembly (650) of claim 1, wherein the device array (676) is coupled to the terminal array (114) through one of a thermo-compression bond, a solderless bond, or an anisotropic conductive film or gel.

8. A system (550) comprising a probe assembly (650) according to claim 1 and a control device (554) for receiving data signals based on the external signals or for transmitting data signals to the modular device (670) for emitting energy.

9. The system (550) of claim 8, wherein the control device (554) includes a display that (556) for displaying information based on the data signals.

10. The system (550) of claim 8, further comprising a probe body (602) for insertion into a body, wherein the probe body (602) surrounds the modular device (670).

11. The system (550) of claim 10, wherein the modular device (670) comprises an ultrasound device (606), preferably wherein the ultrasound device (606) comprises a capacitive micromachined ultrasonic transducer (CMUT) or a piezoelectric micromachined ultrasonic transducers (PMUT).

12. The system (550) of claim 8, wherein the communication lines (656) include wire conductors and conductive bumps (420) directly coupled to corresponding end faces (415) of the wire conductors, the conductive bumps (420) forming corresponding mating terminals (112) and being presented along the mating side (662) to form the terminal array (114), the conductive bumps (420) being electrically coupled to corresponding electrical contacts of the device array (676).

## Patentansprüche

1. Sondenbaugruppe (650), die Folgendes umfasst:
ein modulares Gerät (670), das ein Array von Elementen zum Erkennen externer Signale oder zum Emittieren von Energie umfasst, wobei das modulare Gerät (670) ein Geräte-Array (676) mit elektrischen Kontakten und/oder Lichtwellenleiterenden aufweist, wobei das Array von Elementen kommunikativ mit dem Geräte-Array (676) gekoppelt ist; und
eine Kabelbaugruppe (652) zum kommunikativen Koppeln des modularen Geräts (670) mit einem Computersystem (554) und zum Senden von Datensignalen dadurch, wobei die Kabelbaugruppe (652) einen Array-Verbinder (654) mit einem Verbinderkörper (660) umfasst, der eine Gegenseite (662) aufweist, und Kanäle (246), die durch die Gegenseite (662) und den Verbinderkörper (660) verlaufen, wobei die Kabelbaugruppe (652) mehrere in entsprechenden Kanälen (246) des Verbinderkörpers (660) angeordnete Kommunikationsleitungen (656) beinhaltet, wobei die Kommunikationsleitungen (656) Drahtverbinder und/oder Lichtwellenleiter beinhalten, wobei die Kommunikationsleitungen (656) jeweilige Endflächen (415) haben, die in der Nähe der Gegenseite (662) positioniert sind, um ein Klemmenarray (114) zu bilden, wobei das Klemmenarray (114) mit dem Geräte-Array (676) des modularen Geräts (670) ausgerichtet und gekoppelt ist;
wobei der Verbinderkörper (660) mehrere Substratschichten (120) aufweist, die nebeneinander gestapelt sind und jeweilige Gegenränder (122) aufweisen, die die Gegenseite (662) bilden, wobei die Substratschichten (120) mehrere Schnittstellen (249) bilden, wobei jede Schnittstelle zwischen benachbarten Substratschichten (120) definiert ist, wobei die benachbarten Substratschichten (120) die Kanäle (246) dazwischen definieren.

2. Sondenbaugruppe (650) nach Anspruch 1, die ferner einen Sondenkörper (602) zum Einfügen in einen Körper umfasst, wobei der Sondenkörper (602) das modulare Gerät (670) umgibt.

3. Sondenbaugruppe (650) nach Anspruch 2, wobei das modulare Gerät (670) ein Ultraschallgerät beinhaltet, das einen kapazitiven, mikrobearbeiteten Ultraschallwandler (CMUT) und/oder einen piezoelektrischen mikrobearbeiteten Ultraschallwandler (PMUT) umfasst.

4. Sondenbaugruppe (650) nach Anspruch 1, wobei die Kommunikationsleitungen (656) Drahtleiter und leitfähige Höcker (420) aufweisen, die direkt mit entsprechenden Endflächen (415) der Drahtleiter gekoppelt sind, wobei die leitfähigen Höcker (420) entsprechende Gegenklemmen (112) bilden und entlang der Gegenseite (662) präsentiert werden, um das Klemmenarray (114) zu bilden, wobei die leitfähigen Höcker (420) elektrisch mit entsprechenden elektrischen Kontakten (678) des Geräte-Array (676) gekoppelt sind, wobei vorzugsweise die leitfähigen Höcker (420) eine Höhe haben, die gleich oder kleiner als 100 µm ist, und eine Toleranzgrenze haben, die innerhalb von ± 10 µm liegt.

5. Sondenbaugruppe (650) nach Anspruch 1, wobei die Kanäle (246) geätzte Kanäle (246) und/oder geformte Kanäle (246) sind.

6. Sondenbaugruppe (650) nach Anspruch 1, wobei das Klemmenarray (114) wenigstens 50 Gegenklemmen (112) pro 100 mm² beinhaltet.

7. Sondenbaugruppe (650) nach Anspruch 1, wobei das Geräte-Array (676) mit dem Klemmenarray (114) mit einem aus einer Thermokompressionsbindung, einer lötlosen Bindung und einem anisotropen leitfähigen Film oder Gel gekoppelt ist.

8. System (550), das eine Sondenbaugruppe (650) nach Anspruch 1 und ein Steuergerät (554) zum Empfangen von Datensignalen auf der Basis der externen Signale oder zum Senden von Datensignalen zu dem modularen Gerät (670) zum Emittieren von Energie umfasst.

9. System (550) nach Anspruch 8, wobei das Steuergerät (554) ein Display (556) zum Anzeigen von Informationen auf der Basis der Datensignale beinhaltet.

10. System (550) nach Anspruch 8, das ferner einen Sondenkörper (602) zum Einfügen in einen Körper umfasst, wobei der Sondenkörper (602) das modulare Gerät (670) umgibt.

11. System (550) nach Anspruch 10, wobei das modulare Gerät (670) ein Ultraschallgerät (606) umfasst, wobei das Ultraschallgerät (606) vorzugsweise einen kapazitiven mikrobearbeiteten Ultraschallwandler (CMUT) oder einen piezoelektrischen mikrobearbeiteten Ultraschallwandler (PMUT) umfasst.

12. System (550) nach Anspruch 8, wobei die Kommunikationsleitungen (656) Drahtleiter und leitfähige Höcker (420) aufweisen, die direkt mit entsprechenden Endflächen (415) der Drahtleiter gekoppelt sind, wobei die leitfähigen Höcker (420) entsprechende Gegenklemmen (112) bilden und entlang der Gegenseite (662) präsentiert werden, um das Klemmenarray (114) zu bilden, wobei die leitfähigen Höcker (420) elektrisch mit entsprechenden elektrischen Kontakten des Geräte-Array (676) gekoppelt sind.

## Revendications

1. Assemblage de sonde (650) comprenant :
un dispositif modulaire (670) comprenant un groupe d'éléments pour détecter des signaux externes ou émettre de l'énergie, le dispositif modulaire (670) incluant un groupe de dispositifs (676) possédant au moins un poste parmi des contacts électriques ou des terminaisons de fibres optiques, le groupe d'éléments étant couplé de manière communicative au groupe de dispositifs (676) ; et
un ensemble câbles (652) pour effectuer le couplage de manière communicative du dispositif modulaire (670) à un système informatique (554) et pour transmettre des signaux de données à travers celui-ci, l'ensemble câbles (652) comprenant un connecteur de groupe (654) ayant un corps de connecteur (660) qui inclut un côté d'accouplement (662) et des canaux (246) s'étendant à travers le côté d'accouplement (662) et le corps de connecteur (660), l'ensemble câbles (652) incluant une pluralité de lignes de communication (656) disposées au sein de canaux correspondants (246) du corps de connecteur (660), les lignes de communication (656) incluant au moins un poste parmi des conducteurs filaires ou des fibres optiques, les lignes de communication (656) ayant des faces d'extrémité respectives (415) qui sont positionnées à proximité du côté d'accouplement (662) afin de former un groupe de bornes (114), le groupe de bornes (114) étant aligné avec et couplé au groupe de dispositifs (676) du dispositif modulaire (670) ;
dans lequel le corps de connecteur (660) inclut une pluralité de couches de substrat (120) qui sont empilées côte à côte et ont des bords d'accouplement respectifs (122) qui forment le côté d'accouplement (662), les couches de substrat (120) formant une pluralité d'interfaces (249) dans laquelle chaque interface est définie entre des couches de substrat adjacentes (120), les couches de substrat adjacentes (120) définissant les canaux (246) entre elles.

2. Assemblage de sonde (650) de la revendication 1, comprenant en outre un corps de sonde (602) pour une insertion dans un corps, le corps de sonde (602) entourant le dispositif modulaire (670).

3. Assemblage de sonde (650) de la revendication 2, dans lequel le dispositif modulaire (670) inclut un dispositif ultrasonore qui inclut au moins un poste parmi un transducteur ultrasonique capacitif micro-usiné (CMUT) ou un transducteur ultrasonique piézo-électrique micro-usiné (PMUT).

4. Assemblage de sonde (650) de la revendication 1, dans lequel les lignes de communication (656) incluent des conducteurs filaires et des bossages conducteurs (420) directement couplés à des faces d'extrémité correspondantes (415) des conducteurs filaires, les bossages conducteurs (420) formant des bornes d'accouplement correspondantes (112) et étant présentés le long du côté d'accouplement (662) afin de former le groupe de bornes (114), les bossages conducteurs (420) étant couplés électriquement à des contacts électriques correspondants (678) du groupe de dispositifs (676), de préférence les bossages conducteurs (420) ayant une hauteur qui est inférieure ou égale à 100 µm et ayant une limite de tolérance qui se situe dans des limites de ±10 µm.

5. Assemblage de sonde (650) de la revendication 1, dans lequel les canaux (246) sont au moins un poste parmi des canaux gravés (246) ou des canaux moulés (246).

6. Assemblage de sonde (650) de la revendication 1, dans lequel le groupe de bornes (114) inclut au moins 50 bornes d'accouplement (112) par 100 mm².

7. Assemblage de sonde (650) de la revendication 1, dans lequel le groupe de dispositifs (676) est couplé au groupe de bornes (114) par l'intermédiaire d'un moyen parmi un liaisonnement par thermo-compression, un liaisonnement sans soudure, ou un gel ou un film conducteur anisotrope.

8. Système (550) comprenant un assemblage de sonde (650) selon la revendication 1 et un dispositif de commande (554) pour recevoir des signaux de données sur la base des signaux externes ou pour transmettre des signaux de données au dispositif modulaire (670) pour émettre de l'énergie.

9. Système (550) de la revendication 8, dans lequel le dispositif de commande (554) inclut un afficheur (556) pour afficher des informations sur la base des signaux de données.

10. Système (550) de la revendication 8, comprenant en outre un corps de sonde (602) pour une insertion dans un corps, le corps de sonde (602) entourant le dispositif modulaire (670).

11. Système (550) de la revendication 10, dans lequel le dispositif modulaire (670) comprend un dispositif ultrasonore (606), de préférence le dispositif ultrasonore (606) comprenant un transducteur ultrasonique capacitif micro-usiné (CMUT) ou un transducteur ultrasonique piézo-électrique micro-usiné (PMUT).

12. Système (550) de la revendication 8, dans lequel les lignes de communication (656) incluent des conducteurs filaires et des bossages conducteurs (420) directement couplés à des faces d'extrémité correspondantes (415) des conducteurs filaires, les bossages conducteurs (420) formant des bornes d'accouplement correspondantes (112) et étant présentés le long du côté d'accouplement (662) afin de former le groupe de bornes (114), les bossages conducteurs (420) étant couplés électriquement à des contacts électriques correspondants du groupe de dispositifs (676).
